# EUROPÄISCHE PATENTSCHRIFT

(11) **EP 4 074 187 B1**
(45) Veröffentlichungstag und Bekanntmachung des Hinweises auf die Patenterteilung: **02.04.2025**
(21) Anmeldenummer: 21168920.3
(22) Anmeldetag: 16.04.2021
(51) Int. Cl.: A23J 1/00, A23J 1/10, A23J 3/34, A23K 20/142, A23K 20/147, B01D 9/00, B01D 21/00, A23K 10/26

(54) **VERFAHREN ZUR HERSTELLUNG VON PEPTONEN UMFASSEND PROTEINE- UND AMINOSÄUREN AUS SCHLEIMHÄUTEN VON SCHLACHTTIEREN UND ERHALTENER FILTERKUCHEN**
METHOD FOR THE PREPARATION OF PEPTONES COMPRISING PROTEIN AND AMINO ACIDS FROM MUCOUS MEMBRANES OF SLAUGHTERED ANIMALS AND THE FILTER CAKE OBTAINED
PROCÉDÉ DE PRÉPARATION DE PEPTONES COMPRENANT DES PROTÉINES ET DES ACIDES AMINÉS DE MEMBRANES MUQUEUSES D'ANIMAUX ABATTUS ET GÂTEAU DE FILTRATION OBTENU

(43) Veröffentlichungstag der Anmeldung: 19.10.2022
(73) Patentinhaber: Cremer Oleo GmbH & Co. KG, 20095 Hamburg (DE)
(72) Erfinder: MAINZER, Steffen, 20095 Hamburg (DE); KLEINE KLAUSING, Dr., Heinrich, 40221 Düsseldorf (DE)
(74) Vertreter: RGTH

(56) Entgegenhaltungen:
- WO-A1-94/12524
- GB-A- 2 118 559

## Beschreibung

### Einleitung

Die Erfindung betrifft ein Verfahren zur Herstellung eines Peptons.

### Stand der Technik

Heparin ist ein Wirkstoff, welcher in der Medizin vorwiegend als Antikoagulans eingesetzt wird. Der überwiegende Teil des industriell hergestellten Heparins wird aus der Darmschleimhaut von Schweinen gewonnen. In einem weithin verwendeten Verfahren wird eine Lösung umfassend Inhaltsstoffe der Darmschleimhaut von Schweinen einer enzymatischen Hydrolyse unterzogen. Das in der Lösung enthaltene Heparin wird anschließend an einem Harz adsorbiert, welches dann von der flüssigen Phase abgetrennt wird. Die verbleibende flüssige Phase ist ein Abfallprodukt und enthält neben den Aminosäuren und Peptiden, die bei der enzymatischen Hydrolyse entstehen, noch andere Inhaltstoffe der Darmschleimhaut und Salze, die vor und/oder während der Durchführung des Extraktionsverfahrens in die flüssige Phase eingebracht werden. Bei diesen Salzen handelt es sich in der Regel um Natriumbisulfid, welches zur Stabilisierung der Lösung gegenüber mikrobiellem Wachstum und Verderb eingesetzt wird. Auch andere Salze können in dieser flüssigen Phase enthalten sein. Die flüssige Phase kann wegen ihres hohen Salzgehaltes als solches nicht als Futtermittel weiterverwendet werden. In Futtermitteln führt der hohe Salzgehalt bei den Tieren zu Verdauungsstörungen und als deren Folge können Zuwachsverluste auftreten. Das Eluat fällt daher als Abfallstoff an, obwohl es wertvolle Aminosäuren und Proteine enthält. Es verursacht bei seiner Entsorgung erhebliche Kosten. Daher wird dieser Abfallstoff üblicherweise zu einem Pepton aufbereitet, was unter anderem das Entfernen des Wassers und der Salze beinhaltet. Peptone sind Gemische, welche die Hydrolysate von Proteinen enthalten, also Aminosäuren und Peptide. Die so erhaltenen Peptone enthalten einen hohen Anteil an Aminosäuren und Peptiden der Darmschleimhaut und können als Zusatz zu Futtermitteln und dergleichen verwendet werden.

In der chinesischen Offenlegungsschrift CN 106035980 A wird ein Verfahren beschrieben, in dem das Eluat der Heparinherstellung einer Filtration mit einer Mikrofiltrationsmembran oder Ultrafiltrationsmembran unterworfen wird. Das Permeat wird durch Erhitzen und Destillation weitgehend von Wasser befreit, wodurch der größte Teil der darin enthaltenen Salze auskristallisiert. Das Permeat wird dann mit dem flüssigen Retenat der Mikrofiltration vereinigt und das Gemisch getrocknet. Das so erhaltene Pepton wird nach Zusatz von Hilfsstoffen als Futtermittel und dergleichen verwendet. Dieses Verfahren ist durch das Mikrofiltrationsverfahren sehr aufwändig.

In der WO 2012/069513 A1 wird ein Verfahren zur Aufarbeitung des Eluats aus der Heparinherstellung vorgestellt, in dem zunächst eine Ultrafiltration durchgeführt wird und anschließend die Salze durch eine Elektrodialyse entfernt werden. Auch dieses Verfahren ist durch diese beiden Verfahrensschritte sehr aufwändig und kostenintensiv. Es verbleibt also das Problem, eine kostengünstigere Aufbereitung oder Verwendung für die Abfälle aus der Heparin-Herstellung nach dem enzymatischen Verfahren und andere ähnliche Abfälle aufzufinden.

Aus der WO 94/12524 A1 ist ein Proteinhydrolysat mit typischen Eigenschaften und einem charakteristischen Aminosäureprofil sowie ein Verfahren zu seiner Herstellung und Verwendung. Das Proteinhydrolysat eignet sich als Proteinstreckungsmittel in Tierfutter- und Humanernährungsformulierungen sowie als Stickstoffquelle zur Verwendung in Kulturmedien und als Appetitanreger. Das Proteinhydrolysat wird aus tierischem Gewebe mit einer Endothel- oder Schleimhautkomponente gewonnen. Aus einer Lösung von tierischem Gewebe werden unter nicht-proteolytischen Bedingungen bestimmte Bestandteile entfernt. Die resultierende Rohmateriallösung wird dann mit einem proteolytischen Enzym verdaut, und anschließend werden anionische Verunreinigungen aus der Verdauungslösung entfernt.

Aus der GB 2 118 559 ist ein Verfahren zur Extraktion von Protein und Chrom aus chromgegerbten Hautabfällen bekannt.

### Aufgabe der Erfindung

Der Erfindung liegt die Aufgabe zugrunde, ein Verfahren bereitzustellen, mit dem Abfälle, die große Mengen von anorganischen Salzen und Proteinen und gegebenenfalls Aminosäuren enthalten, kostengünstig, aufbereitet werden können, insbesondere zu Futtermitteln oder Futtermittelzusätzen. Bevorzugt handelt es sich dabei um Abfälle aus der Heparin-Herstellung. Insbesondere ist es eine Aufgabe der vorliegenden Erfindung, ein Verfahren zur Verfügung zu stellen, welches in der Lage ist, aus bei der Heparin-Herstellung anfallenden Abfallstoffen mit einfachen und kostengünstigen Verfahrensschritten ein Pepton herzustellen. Das Pepton soll bevorzugt in der Tierernährung einsetzbar sein. Hierfür sollte das Pepton einen möglichst geringen Anteil an anorganischen Salzen enthalten. Ferner soll das Pepton bevorzugt einen möglichst hohen Anteil an Aminosäuren und Proteinen enthalten.

### Beschreibung der Erfindung

Die vorliegende Erfindung betrifft ein Verfahren zur Aufarbeitung von Zusammensetzungen, die Gemische von Proteinen, Aminosäuren und Salzen enthalten und deren Proteine und Aminosäuren wenigstens teilweise aus Schleimhäuten von Schlachttieren stammen. Es handelt sich um ein Verfahren umfassend die folgenden Schritte:
(1) Bereitstellen einer Zusammensetzung enthaltend Proteine und Aminosäuren aus Schleimhäuten von Schlachttieren, wobei die Schleimhäute von Schlachttieren mit Salzen versetzt und einer enzymatischen Hydrolyse unterzogen wurden, anorganische Salze und Wasser, wobei die Summe der Masse der Proteine und Aminosäuren in der Trockenmasse dieser Zusammensetzung 30 bis 70 Gewichtsprozente beträgt und die Masse der anorganischen Salze in der Trockenmasse dieser Zusammensetzung wenigstens 7,5 Gewichtsprozente beträgt, wobei wenigstens ein Anteil von 50 Gewichtsprozenten der anorganischen Salze aus den Elementen Natrium und Schwefel sowie Chlorid besteht, wobei der Anteil von Natriumsulfat an der Gesamtmenge der anorganischen Salze wenigstens 50 Gewichtsprozente beträgt,
(2) Filtrieren der Zusammensetzung unter Gewinnung eines Filtrats und eines Filterkuchens, wobei zum Filtrieren der Zusammensetzung ein Filter mit einer Porengröße von mehr als 2 µm verwendet wird,
(3) Kühlen des Filtrats auf eine Temperatur im Bereich von -15°C bis 15°C unter Bildung eines Niederschlags,
(4) Abtrennen des entstandenen Niederschlags unter Gewinnung der flüssigen Phase als Produkt.

Durch dieses Verfahren wird mit dem in Schritt (4) abgetrennten Niederschlag ein Teil der in der bereitgestellten Zusammensetzung enthaltenen anorganischen Salze von den ebenfalls in der der bereitgestellten Zusammensetzung enthaltenen Proteinen und Aminosäuren abgetrennt. Die Begriffe "Peptid" und "Protein" werden im Folgenden mit identischer Bedeutung verwendet, das heißt mit beiden Begriffen werden Oligoaminosäuren und Polyaminosäuren bezeichnet, die zwei oder mehr Aminosäurereste enthalten. Das erfindungsgemäße Verfahren ist sehr einfach und kann preiswert und mit einer hohen Raum-Zeit-Ausbeute durchgeführt werden. Es ist dadurch dem Stand der Technik überlegen. Das eigentliche Abtrennen der anorganischen Salze erfolgt durch Kühlen, Bilden eines Niederschlags und dessen Abtrennung. Der Verfahrensschritt (2) des Filtrierens der eingesetzten (bereitgestellten) Zusammensetzung dient der Vorbereitung (Aufbereitung) der Zusammensetzung und ermöglicht erst das Auskristallisieren der anorganischen Salze durch Kühlung.

Vorversuche haben ergeben, dass es nicht möglich ist, aus den in Schritt (1) eingesetzten Zusammensetzungen die anorganischen Salze durch Kühlen direkt auszukristallisieren. Jedenfalls gelingt dies nicht in einer Menge und Geschwindigkeit, die für einen industriellen Prozess notwendig sind. Es wird angenommen, dass solche erfindungsgemäßen Zusammensetzungen Verbindungen enthalten, die die Kristallisation hemmen. Dabei könnte es sich um Polyanionen handeln, wie stark sulfonierte Polysaccharide, die in Schleimhäuten vorkommen. Auch die teilweise abgebauten Proteine aus den Schleimhäuten könnten einen solchen Effekt haben. Überraschenderweise ist es jedoch problemlos möglich, aus diesen Zusammensetzungen nach einer Abtrennung der Feststoffe durch Filtration in Schritt (2) anorganische Salze in Schritt (3) mit hoher Raum-Zeit-Ausbeute auszukristallisieren. Erst dadurch wird das erfindungsgemäße Verfahren kommerziell anwendbar.

Der hohe Anteil an anorganischen Salzen in der in Schritt (1) bereitgestellten Zusammensetzung verhindert die kommerzielle Nutzung der darin enthaltenen Proteine und Aminosäuren als Zusatz zu Futtermitteln und dergleichen. Wenn solche Zusammensetzungen nicht aufgearbeitet werden können, müssen sie mit hohem Aufwand entsorgt werden. Dabei sind die Kosten für die Aufarbeitung entscheidend für den kommerziellen Nutzen des Verfahrens. Alle drei in diesem Verfahren gewonnenen Stoffe, der Filterkuchen aus Schritt (1), der in Schritt (4) abgetrennte Niederschlag und die in Schritt (4) erhaltene flüssige Phase enthalten sowohl Proteine und Aminosäuren als auch anorganische Salze. Der Niederschlag enthält jedoch einen weit höheren Anteil an anorganischen Salzen als die anderen beiden Stoffzusammensetzungen. Durch die anorganischen Salze des im Schritt (3) gebildeten Niederschlags wird der in Schritt (1) bereitgestellten Zusammensetzung ein Teil der anorganischen Salze entzogen. Überraschender Weise enthält auch der in Schritt (2) abgetrennte Filterkuchen deutlich weniger anorganische Salze als die in Schritt (1) eingesetzte Zusammensetzung. In der Regel liegt der Anteil der anorganischen Salze in der Trockenmasse des Filterkuchens aus Schritt (2) und in der Trockenmasse der in Schritt (4) erhaltenen flüssigen Phase unabhängig voneinander wenigstens 25 Gewichtsprozente unter dem Anteil in der Trockenmasse der anorganischen Salze in der in Schritt (1) bereitgestellten Zusammensetzung. Bevorzugt ist der Anteil der anorganischen Salze in der Trockenmasse der in Schritt (4) erhaltenen flüssigen Phase wenigstens 35 Gewichtsprozente und besonders bevorzugt wenigstens 40 Gewichtsprozente geringer. So weit nicht anders erwähnt oder aus dem Kontext erkennbar, beziehen sich alle Mengenangaben in diesem Text auf die Trockenmasse des jeweiligen Stoffes. Für die Zwecke der vorliegenden Erfindung wird der Anteil der anorganischen Salze eines Stoffes durch die Messung des Rohaschegehalts bestimmt.

Mit dem in Schritt (2) anfallenden Filterkuchen und der in Schritt (4) anfallenden flüssigen Phase fallen zwei direkt verwendbare Zusammensetzungen als Verfahrensprodukte an. Sie enthalten deutlich weniger anorganische Salze als die bereitgestellte Zusammensetzung und können daher in der Regel ohne weitere Verarbeitung verwendet werden. Der in Schritt (2) anfallende Filterkuchen und insbesondere die in Schritt (4) anfallende flüssige Phase können beispielsweise in Futtermischungen für Monogastrier sowie auch in Futtermischungen für Hunde, Katzen und Fische eingesetzt werden. Bevorzugt werden den vorgenannten Futtermischungen insgesamt bis zu 5 Gewichtsprozente dieser Verfahrensprodukte zugesetzt, wobei auch hier die Mengen auf die Trockenmasse der Verfahrensprodukte berechnet werden. Die Verfahrensprodukte sind wegen des hohen Protein- und Aminosäuregehalts sehr nahrhaft und daher wertvolle Futtermittelzusätze. Durch den geringen Salzgehalt treten keine unerwünschten Nebeneffekte beim Verfüttern auf. Der entstandene Niederschlag kann in der Regel ebenfalls weiterverwendet werden. Handelt es sich beispielsweise um einen vorwiegend Natriumsulfat enthaltenden Niederschlag, so wird dieses, gegebenenfalls nach einem weiteren Reinigungsschritt, als solches weiterverwendet. Das vorliegende Verfahren eignet sich besonders für Zusammensetzungen die einen großen Anteil an Natriumsulfat in den anorganischen Salzen der in Schritt (1) bereitgestellten Zusammensetzungen enthalten.

Die Verfahrensprodukte können getrocknet werden, was insbesondere zum Transport und zur Lagerung vorteilhaft sein kann. Der in Schritt (2) anfallende Filterkuchen kann durch jede beliebige Trocknungsmethode für Feststoffe getrocknet werden. Die als Endprodukt anfallende flüssige Phase kann durch jede dem Fachmann bekannte Methode zur Trocknung von Flüssigkeiten getrocknet werden, wird jedoch bevorzugt durch Sprühtrocknung getrocknet.

Schleimhäute von Schlachttieren können in der Regel nicht als Nahrungsmittel vertrieben werden. Die anfallenden Mengen können auch nicht vollständig als Zusätze zu Viehfutter, Hundefutter und dergleichen verwendet werden. Eine Verwendung finden sie in der Gewinnung von Inhaltsstoffen wie Heparin und anderen sulfonierten Polysacchariden und Polyaminosacchariden. Auch zur Entsorgung der Schleimhäute unter Gewinnung von Proteinkonzentraten können Schleimhäute aus Schlachttieren einer Hydrolyse der darin enthaltenen Proteine unterzogen werden. Dabei wird in der Regel die Darmschleimhaut von Schlachttieren verwendet, da diese in großen Mengen anfällt und relativ leicht gewonnen werden kann. Die im vorliegenden Verfahren bereitgestellte Zusammensetzung enthält daher bevorzugt Proteine und Aminosäuren aus der Darmschleimhaut von Schlachttieren. Besonders bevorzugt ist Darmschleimhaut von Schweinen, da sie in genügend großen Mengen preiswert zur Verfügung steht. Darmschleimhaut von Rindern wird wegen der - wenn auch nur noch theoretisch vorhandenen - Gefahr der bei Rindern auftretenden bovinen spongiformen Enzephalopathie (BSE) bevorzugt nicht verwendet. Aus demselben Grund werden in Schritt (1) des erfindungsgemäßen Verfahrens bevorzugt Zusammensetzungen eingesetzt, die ausschließlich Proteine enthalten, die eine Masse von weniger als 10.000 Dalton aufweisen. Die Messung der Masse erfolgt dabei mittels Matrix-Assistierter Laser-Desorption-Ionisierung mit Flugzeitanalyse (MALDI-TOF). Besonders bevorzugt sind erfindungsgemäße Verfahren, die dadurch gekennzeichnet sind, dass die in Schritt (1) bereitgestellte Zusammensetzung Proteine und Aminosäuren enthält, welche bei der Extraktion von Heparin aus der Darmschleimhaut von Schlachttieren als Abfallprodukt anfallen. Ferner werden in Schritt (1) des erfindungsgemäßen Verfahrens bevorzugt Zusammensetzungen eingesetzt, die Proteine und Aminosäuren enthalten, die bei einem Verfahren zur Herstellung von Heparin anfallen, bei dem die Darmschleimhaut von Schlachttieren einer enzymatischen Hydrolyse unterzogen wird. Bevorzugterweise werden auch andere Schleimhäute von Schlachttieren, die mit Salzen versetzt und einer enzymatischen Hydrolyse unterzogen wurden, eingesetzt.

Die Filtration der eingesetzten Zusammensetzung in Schritt (2) kann prinzipiell mit jeder geläufigen Methode der Filtration erfolgen. Die Filtrationsmethode kann zum Beispiel ausgesucht werden aus der Gruppe bestehend aus Kammerfilter-Filtration, Mikrofiltration, Vakuum-Filtration, Ultrafiltration, Filtration durch Membranfilterpresse mit oder ohne Nachpresseinrichtung. Auch andere Filtrationsmethoden können verwendet werden, insbesondere solche, bei denen die Filtration durch Anwendung von Druck oder Vakuum unterstützt wird.

Bevorzugt wird zum Filtrieren in Schritt (2) eine Mikrofiltrationsanlage oder eine Kammerfilterpresse verwendet. Besonders bevorzugt ist die Verwendung einer Kammerfilterpresse. Die Kammerfilterpresse bietet viele Vorteile gegenüber der Mikrofiltration, sie ist weniger wartungsintensiv, die Anschaffungskosten sind deutlich geringer und sie weist zudem einen deutlich höheren Durchlauf auf. Zudem ist die Ausbeute bei der Filtration mit einer Kammerfilterpresse sehr hoch. In der Regel werden wenigstens 70 Gewichtsprozente Filtrat erhalten, bezogen auf die in Schritt (1) eingesetzte Zusammensetzung, oft sogar wenigstens 80 Gewichtsprozente. Bevorzugt wird bei einer Temperatur im Bereich von 40 bis 80 °C filtriert.

Bei Anwendung einer Mikrofiltration wird bevorzugt eine keramische Mikrofiltrationseinheit verwendet. Keramische Mikrofiltrationseinheiten sind besonders temperaturbeständig. Insbesondere kann die Mikrofiltration durch Dynamische-Cross-Flow-Filtration mit rotierenden Scheiben durchgeführt werden. Dies ist bevorzugt. Die Porenweite liegt bevorzugt im Bereich von 0,05 bis 1 µm. Der Filtrationsdruck liegt bevorzugt im Bereich von 0,5 bis 5 bar. Die Filtration in Schritt (2) erfolgt bevorzugt bei einer Temperatur im Bereich von 30 bis 80°C.

Bei Verwendung einer Kammerfilterpresse kann jede Art von Kammerfilterpresse verwendet werden. Bevorzugt wird eine Membranfilterpresse mit Nachpresseinheit und Spülfunktion verwendet werden. Hierdurch kann die Ausbeute verbessert und Gehalt der Trockenmasse im Filterkuchen erhöht werden. Für die Filtertücher kann eine Standardqualität verwendet werden.

Eine Vakuum-Filtration kann beispielsweise mit einem Filter ausgesucht aus der Gruppe bestehend aus Bandfilter, Trommelfilter, Scheibenfilter, Tellerfilter und Blattfilter ausgeführt werden.

Die in Schritt (1) bereitgestellte Zusammensetzung enthält bevorzugt 25 bis 50 Gewichtsprozente Proteine und Aminosäuren, 30 bis 60, besonders bevorzugt 35 bis 50, Gewichtsprozente Wasser und 7,5 bis 30 Gewichtsprozente anorganische Salze, jeweils bezogen auf die Gesamtmasse der Zusammensetzung. Die Angabe von "25 bis 50 Gewichtsprozente Proteine und Aminosäuren" ist dabei die Angabe für die Summe aus dem Massenanteil der Proteine und dem Massenanteil der Aminosäuren. Enthält eine Zusammensetzung mehr als 60, bevorzugterweise 50, Gewichtsprozente Wasser, so kann dieser das Wasser durch geeignete Maßnahmen entzogen werden. Daher ist ein erfindungsgemäßes Verfahren bevorzugt, dass dadurch gekennzeichnet ist, dass das Bereitstellen der Zusammensetzung in Schritt (1) das Entfernen von Wasser aus einem Ausgangsstoff umfasst. Entsprechend kann aus Konzentraten durch Hinzufügen von Wasser eine geeignete Zusammensetzung hergestellt werden. Dies entspricht einem erfindungsgemäßen Verfahren, dass dadurch gekennzeichnet ist, dass das Bereitstellen der Zusammensetzung in Schritt (1) das Hinzufügen von Wasser zu einem Ausgangsstoff umfasst.

Solche Zusammensetzungen können aus Abwässern der Heparin-Herstellung und ähnlichen Verfahren durch einfaches Entfernen eines Teils des Wassers hergestellt werden. Abwässer aus der Heparin-Herstellung enthalten für gewöhnlich etwa 80 bis 97 Gewichtsprozente Wasser. Die Entsorgung dieser Abwässer ist wegen der großen Menge außerordentlich teuer. Aufgrund der großen Masse können diese auch nicht einfach transportiert werden. Hersteller von Heparin müssen die Abfälle daher entweder selbst aufarbeiten oder ihnen einen Teil des Wassers entziehen, um den Transport zu einem Abfallverwerter kommerziell attraktiver zu machen. Abwässer aus der Heparin-Herstellung, denen ein Teil des Wassers entzogen wurde und die die direkt vorstehend genannte Zusammensetzung aufweisen, werden bevorzugt im vorliegenden Verfahren eingesetzt. Solche Zusammensetzungen weisen bei 50 °C in etwa eine Viskosität von etwa 20.000 cPs auf. Bei 70 °C beträgt die Viskosität immer noch etwa 8500 cPs, was in etwa der Viskosität von Honig bei Raumtemperatur entspricht. Es ist nicht möglich, aus solchen Zusammensetzungen mit herkömmlichen Verfahren anorganische Salze auszukristallisieren. Dennoch sind solche Zusammensetzungen noch gut filtrierbar. Zur Senkung der Viskosität können die Zusammensetzungen bei der Filtration in Schritt (2) des erfindungsgemäßen Verfahrens erwärmt werden. Bevorzugt werden diese auf eine Temperatur von 30 bis 80 °C erwärmt, besonders bevorzugt auf eine Temperatur von 40 bis 70 °C.

Überraschender Weise weist das in Schritt (2) aus solchen hochviskosen Zusammensetzungen erhaltene Filtrat eine niedrigere Viskosität auf als die in Schritt (1) bereitgestellten Zusammensetzungen. Dies könnte die Kristallisation von anorganischen Salzen aus den in Schritt (2) gewonnenen Filtraten begünstigen. Besonders bevorzugt ist ein erfindungsgemäßes Verfahren in dem die in Schritt (1) bereitgestellte Zusammensetzung 30 bis 45 Gewichtsprozente Proteine und Aminosäuren, 40 bis 50 Gewichtsprozente Wasser und/oder 7,5 bis 25 Gewichtsprozente anorganische Salze umfasst, jeweils bezogen auf die gesamte Masse der Zusammensetzung.

Der beim Filtrieren der in Schritt (1) bereitgestellten Zusammensetzung in Schritt (2) gewonnene Filterkuchen enthält ebenso wie die in Schritt (1) bereitgestellte Zusammensetzung selbst Proteine, Aminosäuren und anorganische Salze, jedoch sind die anorganischen Salze in deutlich geringeren Mengen enthalten. Typischerweise enthält der Filterkuchen so geringe Mengen an anorganischen Salzen, dass er als Proteinzusatz in Tierfutter oder zu anderen Zwecken direkt verwendet werden kann. Der Gehalt an anorganischen Salzen des in Schritt (2) erhaltenen Filterkuchens beträgt weniger als 25 Gewichtsprozente bezogen auf die Trockenmasse des Filterkuchens, bevorzugt weniger als 20 Gewichtsprozente. Bevorzugterweise wird der Filterkuchen zur weiteren Herabsetzung des Gehalts an anorganischen Salzen mit Wasser gewaschen. Der Filterkuchen kann ferner getrocknet werden und er kann gegebenenfalls durch Mahlen zu einem Pulver weiterverarbeitet werden.

Filter mit kleinen Porengrößen verstopfen schnell bei der Filtration in Schritt (1). Der Grund dafür ist wahrscheinlich, dass in der in Schritt (1) bereitgestellten Zusammensetzung Partikel mit verschiedensten Größen enthalten sind. Dadurch werden die Filterporen durch kleine Partikel zugesetzt und die Filtration behindert. Die dabei auftretenden Komplikationen senken die Raum-Zeit-Ausbeute erheblich. Daher sind Verfahren erfindungsgemäß, die dadurch gekennzeichnet sind, dass zum Filtrieren der Zusammensetzung ein Filter mit einer Porengröße von mehr als 2 µm verwendet wird, bevorzugt mehr als 10 µm. Aus diesem Grunde kann es bevorzugt sein in Schritt (2) keine Mikrofiltration zu verwenden.

Während es sehr schwierig oder nahezu unmöglich ist, die in der eingesetzten Zusammensetzung enthaltenen anorganischen Salze auszukristallisieren, zeigen die anorganischen Salze in dem in Schritt (2) gewonnenen Filtrat überraschenderweise eine deutlich ausgeprägte Neigung zum Kristallisieren. Um die anorganischen Salze in genügender Menge und Geschwindigkeit zur Kristallisation zu bringen muss das Filtrat jedoch gekühlt werden. Wie vorstehend beschrieben, wird das Filtrat auf eine Temperatur im Bereich von -15 bis 15 °C gebracht. Geringere Temperaturen führen zur Gelierung des Filtrats oder zum Auskristallisieren von Wasser. Beides verlangsamt das Kristallisieren der anorganischen Salze oder unterbindet es vollständig, außerdem wird verhindert, dass ein eventuell entstandener Niederschlag effektiv abgetrennt werden kann. Höhere Temperaturen führen zu keiner Kristallisation oder nur zu einer geringen Menge an Niederschlag. Bevorzugt ist ein erfindungsgemäßes Verfahren, welches dadurch gekennzeichnet ist, dass das in Schritt (2) erhaltene Filtrat zur Bildung des Niederschlags in Schritt (3) auf eine Temperatur im Bereich von -12,5 bis 12,5 °C und besonders bevorzugt im Bereich von -10°C bis 10 °C gekühlt wird. Am meisten bevorzugt sind -5 bis 5 °C. Dies führt zu der größtmöglichen Abtrennung von anorganischen Salzen mit einer hohen Raum-Zeit-Ausbeute. Die Kristallisation ist in der günstigsten Ausführungsform in einem diskontinuierlichen Verfahren bei Temperaturen von -5 bis 5 °C in etwa 1,5 bis 3 Stunden abgeschlossen. Die Kristallisation wird beendet, wenn weitere Kristalle nur noch in geringer Menge gebildet werden. Die günstigsten Bedingungen in Bezug auf Temperatur und Zeitdauer kann der Fachmann leicht und gegebenenfalls unter Berücksichtigung von Ausbeute und Energieaufwand durch Vorversuche bestimmen.

Die Kristallisation kann durch übliche Methoden unterstützt werden, zum Beispiel durch Zugabe von Impfkristallen oder durch Einbringen von Energie, zum Beispiel durch Rühren, Reiben mit einem Glasstab usw. Als Impfkristalle eignen sich zum Beispiel Aktivkohle oder Natriumsulfat-Kristalle und zwar auch solche wie sie beim erfindungsgemäßen Verfahren nach Trocknung des Niederschlags aus Schritt (4) des erfindungsgemäßen Verfahrens anfallen. Die Zugabe von Impfkristallen erfolgt bevorzugt bei einer Temperatur von 5 bis 15 °C und die Impfkristalle sind ebenfalls bevorzugt auf eine Temperatur in diesem Bereich vorgekühlt.

### Vorbereitung der Kristallisation

Stellt sich heraus, dass das gemäß Schritt (2) erhaltene Filtrat eine für die Kristallisation ungünstige Konzentration an anorganischen Salzen aufweist, so kann diese für die folgende Kristallisation gemäß Schritt (3) des erfindungsgemäßen Verfahrens neu eingestellt werden. Bevorzugt ist daher ein erfindungsgemäßes Verfahren, das dadurch gekennzeichnet ist, dass vor dem Kühlen des Filtrats, dessen Wassergehalt auf eine für die Kristallisation günstige Konzentration gebracht wird. Ist die Konzentration der anorganischen Salze und damit auch die Konzentration der Proteine und/oder Aminosäuren zu hoch, so kann das Filtrat mit Wasser verdünnt werden. Weist das Filtrat eine für die Kristallisation zu geringe Konzentration an anorganischen Salzen auf, so können diese durch Entzug von Wasser aufkonzentriert werden. Bevorzugt geschieht dies durch Verdampfung des Wassers. Membranverfahren sind ebenfalls verwendbar, sind jedoch in der Regel zu aufwändig. Ob die Konzentration der Salze im Filtrat für die Kristallisation günstig ist, kann leicht durch Vorversuche geprüft werden. Wenn jedoch die in Schritt (1) eingesetzte Zusammensetzung die vorstehend als bevorzugt genannten Gehalte an Proteinen und Aminosäuren, anorganischen Salzen und Wasser enthält, weist das Filtrat eine für die Kristallisation günstige Konzentration auf.

Bevorzugt enthält das in Schritt (2) erhaltene Filtrat 25 bis 50 Gewichtsprozente Proteine und Aminosäuren, 30 bis 60 Gewichtsprozente Wasser und 10 bis 30 Gewichtsprozente anorganische Salze, jeweils bezogen auf die Gesamtmasse des Filtrats. Besonders bevorzugt enthält das in Schritt (2) erhaltene Filtrat 30 bis 45 Gewichtsprozente Proteine und Aminosäuren, 40 bis 55 Gewichtsprozente Wasser und 10 bis 25 Gewichtsprozente anorganische Salze, jeweils bezogen auf die Gesamtmasse des Filtrats.

Der in Schritt (4) gebildete Niederschlag kann durch jede bekannte Methode von der Mutterlauge befreit werden. Bevorzugt sind jedoch Dekantieren, Zentrifugieren oder Filtrieren. Da die Mutterlauge in der Regel hochviskos ist, haftet dem Niederschlag eine große Menge der Mutterlauge an. Sie kann am gründlichsten durch Zentrifugation entfernt werden. Bevorzugt ist daher ein erfindungsgemäßes Verfahren, welches dadurch gekennzeichnet ist, dass die Abtrennung des in Schritt (3) gebildeten Niederschlags in Schritt (4) durch Zentrifugation erfolgt. Am meisten bevorzugt ist eine Kombination aus Dekantieren und Zentrifugieren oder aus Filtrieren und Zentrifugieren. Dadurch gelingt es, die Mutterlauge einfach, schnell und gründlich vom Niederschlag abzutrennen.

Die im erfindungsgemäßen Verfahren eingesetzten (bereitgestellten) Zusammensetzungen enthalten in der Regel als Anionen Sulfat und Chlorid in größeren Mengen, wobei Sulfate in der Regel überwiegen. Daneben liegen häufig Sulfite und Phosphate in kleineren Mengen vor. Als Kationen treten überwiegend Natrium und Kalium auf, wobei wiederum Natrium in der Regel weit häufiger ist. In geringfügigen Mengen sind auch zweiwertige Kationen wie Magnesium und/oder Calcium enthalten. Das vorliegende Verfahren kann für Zusammensetzungen mit beliebigen anorganischen Salzen angewendet werden; erfindungsgemäß ist das Verfahren gemäß Anspruch 1.

Es ist jedoch auf die Kristallisation von Natriumsulfat optimiert. Dies gilt insbesondere für die bevorzugten Ausführungsformen. Entsprechend ist der Einsatz (das Bereitstellen) von erfindungsgemäßen Zusammensetzungen in Schritt (1) vorgesehen, in denen wenigstens ein Anteil von 50 Gewichtsprozenten der anorganischen Salze aus den Elementen Natrium und Schwefel sowie Chlorid besteht. Erfindungsgemäß ist der Einsatz (das Bereitstellen) von Zusammensetzungen in Schritt (1) in denen der Anteil von Natriumsulfat an der Gesamtmenge der anorganischen Salze wenigstens 50 Gewichtsprozente beträgt, bevorzugt sind wenigstens 70 Gewichtsprozente.

Entsprechend bestehen in der Regel auch wenigstens 50 Gewichtsprozente des in Schritt (4) gewonnenen Niederschlags aus anorganischen Salzen der Elemente Natrium und Schwefel sowie Chlorid. Hiermit ist gemeint, dass mindestens 50 Gewichtsprozente der Rohasche aus der Summe der Gewichtsprozente der Elemente Natrium, Schwefel sowie Chlorid bestehen, wobei die Gehalte der Rohasche, Natrium, Schwefel und Chlorid durch die nachstehend benannten Messmethoden bestimmt werden können. Bevorzugt bestehen wenigstens 50 Gewichtsprozente des in Schritt (4) gewonnenen Niederschlags aus Sulfaten, besonders bevorzugt wenigstens 70 Gewichtsprozente. Hiermit ist gemeint, dass mindestens 50, besonders bevorzugt wenigstens 70 Gewichtsprozente der Rohasche aus der Summe der Gewichtsprozente der Sulfate bestehen, wobei der Gehalt der Sulfate durch die nachstehend benannte Messmethode bestimmt werden kann. Der Niederschlag enthält daneben auch Proteine, Aminosäuren, Wasser und andere Salze. Der Niederschlag kann mit wenig Wasser oder Mutterlauge gespült werden. Das Spülwasser kann verworfen oder dem Verfahren erneut hinzugefügt werden.

Bei dem erfindungsgemäßen Verfahren werden in der Regel mindestens zwei, insbesondere genau zwei Produkte erhalten, nämlich die in Schritt (2) erhaltene Filterkuchen und die in Schritt (4) abgetrennte flüssige Phase. Die Produkte des erfindungsgemäßen Verfahrens sind Peptone. Bevorzugt ist ein erfindungsgemäßes Verfahren, dass dadurch gekennzeichnet ist, dass die in Schritt (4) erhaltene flüssige Phase und/oder der in Schritt (2) erhaltene Filterkuchen weniger als 25 Gewichtsprozente anorganischer Salze, besonders bevorzugt weniger als 20 Gewichtsprozente anorganischer Salze, ganz besonders bevorzugt weniger als 17,5 Gewichtsprozente anorganischer Salze bezogen auf die Trockenmasse enthält. Diese Mengenangaben gelten für die in Schritt (4) erhaltene flüssige Phase und für den in Schritt (2) erhaltenen Filterkuchen jeweils unabhängig voneinander. Das vorliegende Verfahren verringert den Anteil an anorganischen Salzen in den Produkten jeweils gegenüber der eingesetzten Zusammensetzung. Bevorzugt ist ein erfindungsgemäßes Verfahren, dass dadurch gekennzeichnet ist, dass der Anteil an anorganischen Salzen an der Trockenmasse der in Schritt (4) erhaltenen flüssigen Phase und/oder an der Trockenmasse des in Schritt (2) erhaltenen Filterkuchens gegenüber dem Anteil der anorganischen Salze an der Trockenmasse der eingesetzten Zusammensetzung um wenigstens 25%, bevorzugt um wenigstens 40% und am meisten bevorzugt um wenigstens 45% verringert wird.

Beansprucht wird ein Verfahren gemäß Ansprüchen 1-11.

Ein weiterer Gegenstand der vorliegenden Erfindung ist ein Filterkuchen gemäß Anspruch 12, der dadurch gekennzeichnet ist, dass er nach einem erfindungsgemäßen Verfahren hergestellt wurde.

### Beispiele:

### Messmethoden:

Trockensubstanz -> Bestimmung des Feuchtigkeitsgehaltes VO (EG) 152/2009 Anhang III, A; VDLUFA III, 3.1., 2009; ASU F 0001 (EG):2010-09

Rohprotein VO (EG) 152/2009 Anhang III, C; VDLUFA III, 4.1.1, 1993; ASU F 0003 (EG):2010-09

Rohasche VO (EG) 152/2009 Anhang III, M; VDLUFA III, 8.1, 1979; ASU F 0014 (EG): 2010-09

| | |
|---|---|
| Schwefel gesamt: | DIN EN ISO 11885:2009-09 |
| Natrium: | DIN EN ISO 11885:2009-09 |
| Chlorid: | DIN EN ISO 10304-1:2009 |
| Kalium: | DIN EN ISO 11885:2009-09 |
| Phosphor gesamt: | DIN EN ISO 11885:2009-09 |
| Sulfat | Analog DIN 38405-5-2 |

### Beispiel 1: Filtration mittels Kammerfilterpresse

Es wird eine Kammerfilterpresse mit den Plattenmaßen 250 mm mal 250 mm verwendet. Die Kammerfilterpresse ist mit 21 Kammern bestückt, welche ein Gesamtvolumen von 20,9 L haben. Als Filtertuch kommt ein Polypropylen-Gewebe (PP2436) mit einer Maschenweite von 1 - 5 µm zum Einsatz. Insgesamt verfügt die Kammerfilterpresse über eine Filterfläche von ca. 2 m².

Die Filtration wurde bei 60 °C durchgeführt. Es wurden 91 L einer bereitgestellten Zusammensetzung filtriert und 70 L Filtrat erhalten. Dies entspricht einer Ausbeute von 77 Volumenprozenten. Die Filterkammern waren vollständig ausgefüllt. Damit betrug die Menge des Filterkuchens etwa 21 L. Die Filtration dauerte eine Stunde.

Die folgende Tabelle gibt eine Übersicht über einige der Inhaltstoffe der bereitgestellten Zusammensetzung, des Filtrats und des Filterkuchens an.

**Tabelle 1:**

| Wert | bereitgestellte Zusammensetzung | Filtrat* | Filterkuchen* |
|---|---|---|---|
| Wasser | 47,0 | 49,5 | 36,4 |
| Trockenmasse | 53,0 | 50,5 | 63,6 |
| Protein | 62,0 | 61,8 | 63,5 |
| Rohasche | 27,0 | 30,5 (+13%) | 17,6 (-35%) |
| Schwefel | 6,30 | 7,06 (+12%) | 4,24 (-33%) |
| Natrium | 7,70 | 8,65 (+12%) | 4,98 (-35%) |
| Chlorid | 1,53 | 1,68 (+10%) | 1,01 (-34%) |
| Kalium | 1,12 | 1,25 (+12%) | 0,70 (-38%) |
| Phosphor | 0,95 | 1,05 (+11%) | 0,65 (-32%) |

| | | | |
|---|---|---|---|
| Alle Angaben in Massenprozent. Für die Feuchtigkeit und die Trockenmasse bezogen auf die Gesamtmenge der eingesetzten Zusammensetzung. Für alle anderen Werte bezogen auf die Trockenmasse. *In Klammern die Veränderung gegenüber der bereitgestellten Zusammensetzung in Prozent. | | | |

Wie der Tabelle 1 entnommen werden kann, ändert sich der Proteingehalt bei der Filtration kaum. Sowohl in der eingesetzten Zusammensetzung als auch im Filtrat als auch im Filterkuchen sind etwa 62 Gewichtsprozente Protein enthalten. Der Anteil der Rohasche im Filterkuchen ist jedoch um etwa ein Drittel reduziert. Der Massenanteil aller gemessenen Elemente verringert sich im gleichen Maße. Dass der Proteingehalt nur leicht erhöht ist deutet darauf hin, dass sich im Filterkuchen nicht Proteine, sondern andere organische Stoffe angereichert haben. Es wird angenommen, dass es sich dabei um Zuckerseitenketten der Proteine der Darmschleimhäute handelt.

Mit 17 Gewichtsprozenten Gehalt an Rohasche und davon jeweils etwa 4 bzw. 5 Gewichtsprozenten Schwefel- bzw. Natriumgehalt ist der Filterkuchen überraschenderweise direkt als Futterzusatz geeignet.

### Beispiel 2: Kristallisation kontinuierlich

Für die kontinuierliche Kristallisation wurde aus Beispiel 1 erhaltenes Filtrat verwendet. Die Zusammensetzung des verwendeten Filtrats ist in der folgenden Tabelle 2 angegeben. Es wurden 206,9 kg des in Beispiel 1 erhaltenen Filtrats in einem Behälter vorgelegt und zur Kristallisation in eine Gefrierkonzentrationseinheit (W6-9 Prodias von GEA Niro PT B.V.) überführt. Der Niederschlag wurde durch Zentrifugation abgetrennt und die flüssige Phase (Mutterlauge) als Produkt erhalten. Innerhalb von 17 Stunden wurden hierdurch 156 kg flüssige Phase und 34,9 kg Niederschlag erhalten. Hieraus ergibt sich eine Ausbeute von 16,9 Massenprozenten bezogen auf den gewonnenen Niederschlag. 15,8 kg des eingesetzten Filtrats gingen in der Apparatur, durch Anhaften an der Apparatur oder durch Toträume und dergleichen verloren. Die Temperatur des eingesetzten Filtrats wurde kontinuierlich gemessen, um die Kristallisationstemperatur zu bestimmen. Die durchschnittliche Kristallisationstemperatur betrug -1,5 °C. Wobei die Temperatur der Gefrierkonzentrationseinheit zwischen 0 °C und -6 °C schwankte. Die Viskosität des so erhaltenen Filtrats betrug bei einer Temperatur von -1,25 °C 70 cST (84 cP @1200 kg/m³), gemessen mit einem Ostwald Viskosimeter. Die Partikelgröße des Niederschlags ergab eine Verteilung bei der 50% der Partikel über 100 µm Durchmesser aufwiesen. Es wurden diverse Proben während des gesamten Prozesses genommen und die Werte einer repräsentativen Probe sind in Tabelle 2 dargestellt.

**Tabelle 2:**

| Wert | Filtrat aus Beispiel 1 | Flüssige Phase* | Niederschlag* |
|---|---|---|---|
| Wasser | 48,2 | 48,5 | 54,5 |
| Trockenmasse | 51,8 | 51,5 | 45,5 |
| Protein | 63,9 | 75,8 (+19%) | 13,4 (-79%) |
| Rohasche | 31,2 | 14,3 (-54%) | 85,3 (+173%) |
| Schwefel | 6,73 | 3,89 (-42 %) | 18,6 (+176%) |
| Natrium | 8,24 | 3,90 (-53%) | 26,59 (+223%) |
| Chlorid | 1,62 | 2,07 | 0,37 |
| Kalium | 1,22 | 1,57 | 0,29 |
| Phosphor | 1,02 | 1,25 | 0,26 |

| | | | |
|---|---|---|---|
| Alle Angaben in Massenprozent. Für die Feuchtigkeit und die Trockenmasse bezogen auf die Gesamtmenge der eingesetzten Zusammensetzung. Für alle anderen Werte bezogen auf die Trockenmasse. *In Klammern die Veränderung gegenüber der bereitgestellten Zusammensetzung in Prozent. | | | |

Während in der flüssigen Phase der Proteingehalt gesteigert ist, ist der Gehalt an Rohasche um über 50% in Bezug auf das verwendete Filtrat vermindert. Die flüssige Phase kann in dieser Form als Futtermittel verwendet werden. Der Niederschlag hat einen hohen Anteil an Natrium und Schwefel, woraus sich vermuten lässt, dass es sich größtenteils um Natriumsulfat handelt.

### Beispiel 3: Kristallisation diskontinuierlich gemäß Schritt (3)

Ein mit Rührer und Thermometer ausgestattetes Glasgefäß wurde mit einer einzusetzenden Zusammensetzung gefüllt. Bei dieser Zusammensetzung handelt es sich um ein Filtrat, welches durch Mikrofiltration gemäß Schritt (2) des erfindungsgemäßen Verfahrens erhalten wurde. Die genaue Zusammensetzung kann der mit "Filtrat" überschriebenen Spalte der Tabelle 3 entnommen werden. Das Glasgefäß wurde in eine auf -10°C gekühlte Kühlkammer gestellt. Die Zusammensetzung wurde kontinuierlich gerührt. Der Versuch wurde beendet, als der Inhalt des Glasgefäßes eine Temperatur von -2,5°C erreicht hatte. Die Trennung von flüssiger Phase (Überstand) und Kristallen erfolgte mittels Filtration durch einen Büchnertrichter. Der im Büchnertrichter verbliebene Niederschlag wurde im Anschluss zusätzlich mit einer Handzentrifuge von weiterer anhaftender flüssiger Phase befreit. Die beiden Fraktionen der flüssigen Phase wurden vereint. Die folgende Tabelle 3 gibt eine Übersicht über einige der Inhaltstoffe des eingesetzten Filtrats und der so erhaltenen flüssigen Phase an.

**Tabelle 3:**

| Wert | Filtrat | flüssige Phase* |
|---|---|---|
| Protein | 57,6 | 61,3 |
| Rohasche | 33,0 | 11,7 (-64%) |
| Schwefel | 7,11 | 3,64 (-49%) |
| Natrium | 8,42 | 2,84 (-66%) |
| Chlorid | 0,17 | 0,19 |
| Kalium | 0,77 | 1,16 |
| Phosphor | 0,73 | 1,00 |

| | | |
|---|---|---|
| Alle Angaben in Massenprozent bezogen auf die Trockenmasse. *In Klammern die Veränderung gegenüber dem Filtrat in Prozent. | | |

Durch die Kristallisation und das Entfernen des Niederschlags durch Zentrifugation wird ein signifikanter Anteil der anorganischen Bestandteile aus dem in Schritt (2) erhaltenen Filtrat entfernt. Wie Tabelle 3 zu entnehmen ist, werden hauptsächlich Natrium und Schwefel entfernt, was auf ein Kristallisieren von hauptsächlich Natriumsulfat hindeutet.

Mit einem Gehalt an Rohasche von nur 11,7 Gewichtsprozenten, 3,64 Gewichtsprozenten Schwefel und 2,84 Gewichtsprozenten Natrium ist das Filtrat direkt verwendbar, zum Beispiel als Futtermittelzusatz.

Ein unter genau gleichen Bedingungen durchgeführter Versuch mit einer nicht filtrierten Zusammensetzung gemäß Schritt (1) des erfindungsgemäßen Verfahrens erbrachte keinen Niederschlag. Auch nicht bei Abkühlen auf -10°C und einer längeren Versuchsdauer. Es wurden bei diesen Versuchen verschiedenste Zusammensetzungen eingesetzt, unter anderem auch die in Beispiel 1 eingesetzte Zusammensetzung. In keinem Fall konnte ein Niederschlag erhalten werden.

Ein Ausführungsbeispiel der Erfindung wird nachstehend anhand einer Zeichnung näher erläutert.

Es zeigt:

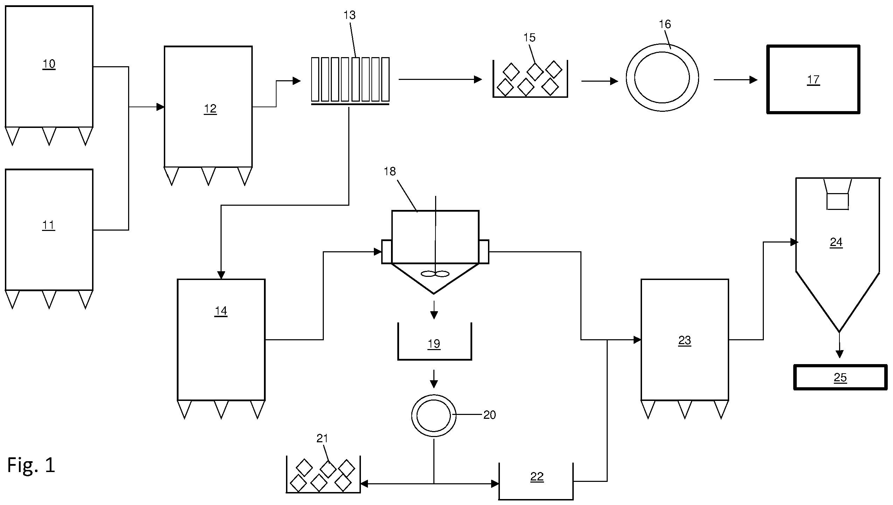

- Fig. 1:: Eine schematische Darstellung des Verfahrens.

In Figur 1 wird eine Übersicht über das Verfahren zur Gewinnung von Peptonen aus Darmschleimhaut dargestellt. Eine Rohlösung 10 wird, wenn nötig, mit Wasser 11 verdünnt und ergibt eine bereitgestellte Zusammensetzung 12. Die bereitgestellte Zusammensetzung 12 wird über eine Kammerfilterpresse 13 filtriert, wobei ein Filtrat 14 und ein Filterkuchen 15 gewonnen werden. Der Filterkuchen 15 wird in einem Walzentrockner 16 getrocknet und ein getrockneter Filterkuchen 17 erhalten.

Das erhaltene Filtrat 14 wird in einem Gefrierkristallisator 18 auf eine Durchschnittstemperatur von ca. -5 °C gekühlt, wobei ein Niederschlag 19 entsteht und von der flüssigen Phase 23 getrennt wird. Der Niederschlag 19 wird über eine Zentrifuge 20 zentrifugiert und der Überstand 22 mit der übrigen flüssigen Phase 23 vereint, während die ausgefallenen Salze 21 getrocknet werden.

Die flüssige Phase 23 wird durch eine Sprühtrocknungsanlage 24 getrocknet, um ein Pepton in Pulverform 25 zu erhalten.

### Bezugszeichenliste:

- 10: Rohlösung
- 11: Wasser
- 12: bereitgestellte Zusammensetzung
- 13: Kammerfilterpresse
- 14: Filtrat
- 15: Filterkuchen
- 16: Walzentrocker
- 17: getrockneter Filterkuchen
- 18: Gefrierkristallisator
- 19: Niederschlag

- 20: Zentrifuge
- 21: ausgefallene Salze
- 22: Überstand
- 23: Flüssige Phase
- 24: Sprühtrocknungsanlage
- 25: Pepton in Pulverform

## Patentansprüche

1. Verfahren umfassend die Schritte:
(1) Bereitstellen einer Zusammensetzung (12) enthaltend Proteine und Aminosäuren aus Schleimhäuten von Schlachttieren, wobei die Schleimhäute von Schlachttieren mit Salzen versetzt und einer enzymatischen Hydrolyse unterzogen wurden, anorganische Salze und Wasser, wobei die Summe der Masse der Proteine und Aminosäuren in der Trockenmasse dieser Zusammensetzung 30 bis 70 Gewichtsprozente beträgt und die Masse der anorganischen Salze in der Trockenmasse dieser Zusammensetzung (12) wenigstens 7,5 Gewichtsprozente beträgt, wobei wenigstens ein Anteil von 50 Gewichtsprozenten der anorganischen Salze aus den Elementen Natrium und Schwefel sowie Chlorid besteht, wobei der Anteil von Natriumsulfat an der Gesamtmenge der anorganischen Salze wenigstens 50 Gewichtsprozente beträgt,
(2) Filtrieren der Zusammensetzung (12) unter Gewinnung eines Filtrats (14) und eines Filterkuchens (15), wobei zum Filtrieren der Zusammensetzung (12) ein Filter mit einer Porengröße von mehr als 2 µm verwendet wird,
(3) Kühlen des Filtrats (14) auf eine Temperatur im Bereich von -15°C bis 15°C unter Bildung eines Niederschlags (19),
(4) Abtrennen des entstandenen Niederschlags (19) unter Gewinnung der flüssigen Phase (23) als Produkt,
wobei zur Bestimmung der Masse an Proteinen und Aminosäuren sowie Salzen die in der Beschreibung genannten Messverfahren eingesetzt werden.

2. Verfahren nach Anspruch 1, **dadurch gekennzeichnet, dass** die in Schritt (1) bereitgestellte Zusammensetzung (12) Proteine und Aminosäuren aus der Darmschleimhaut von Schlachttieren enthält.

3. Verfahren nach einem der Ansprüche 1 oder 2, **dadurch gekennzeichnet, dass** die in Schritt (1) bereitgestellte Zusammensetzung (12) Proteine und Aminosäuren enthält, welche bei der Extraktion von Heparin aus der Darmschleimhaut von Schlachttieren als Abfallprodukt anfallen.

4. Verfahren nach einem der vorangehenden Ansprüche, **dadurch gekennzeichnet, dass** zum Filtrieren der Zusammensetzung (12) in Schritt (2) eine Mikrofiltrationsanlage oder eine Kammerfilterpresse (13) verwendet wird.

5. Verfahren nach einem der vorangehenden Ansprüche, **dadurch gekennzeichnet, dass** die in Schritt (1) bereitgestellte Zusammensetzung (12) 25 bis 50 Gewichtsprozente Proteine und Aminosäuren, 30 bis 60, bevorzugt 35 bis 50, Gewichtsprozente Wasser und 7,5 bis 30 Gewichtsprozente anorganische Salze umfasst.

6. Verfahren nach einem der vorangehenden Ansprüche, **dadurch gekennzeichnet, dass** das in Schritt (2) erhaltene Filtrat (14) zur Bildung des Niederschlags (19) in Schritt (3) auf eine Temperatur im Bereich von -12,5 °C bis 12 °C, bevorzugt von -10 °C bis 10 °C und besonders bevorzugt von -5°C bis 5°C gekühlt wird.

7. Verfahren nach einem der vorangehenden Ansprüche, **dadurch gekennzeichnet, dass** wenigstens 50 Gewichtsprozente des in Schritt (4) erhaltenen Niederschlags (19) aus anorganischen Salzen der Elemente Natrium und Schwefel sowie Chlor bestehen.

8. Verfahren nach einem der vorangehenden Ansprüche, **dadurch gekennzeichnet, dass** die Abtrennung des in Schritt (3) gebildeten Niederschlags (19) in Schritt (4) durch Zentrifugation erfolgt.

9. Verfahren nach einem der vorangehenden Ansprüche, **dadurch gekennzeichnet, dass** die in Schritt (4) erhaltene flüssige Phase (23) weniger als 25 Gewichtsprozente anorganischer Salze, besonders bevorzugt weniger als 20 Gewichtsprozente anorganischer Salze, ganz besonders bevorzugt weniger als 17,5 Gewichtsprozente anorganischer Salze bezogen auf die Trockenmasse enthält.

10. Verfahren nach einem der vorangehenden Ansprüche, **dadurch gekennzeichnet, dass** der in Schritt (2) erhaltene Filterkuchen (15) weniger als 25 Gewichtsprozente anorganischer Salze, besonders bevorzugt weniger als 20 Gewichtsprozente anorganischer Salze bezogen auf die Trockenmasse enthält.

11. Verfahren nach einem der vorangehenden Ansprüche, **dadurch gekennzeichnet, dass** der Anteil an anorganischen Salzen an der Trockenmasse der in Schritt (4) erhaltenen flüssigen Phase (23) und/oder an der Trockenmasse des in Schritt (2) erhaltenen Filterkuchens (15) gegenüber dem Anteil der anorganischen Salze an der Trockenmasse der eingesetzten Zusammensetzung um wenigstens 25%, bevorzugt um wenigstens 35% und am meisten bevorzugt um wenigstens 40% verringert wird.

12. Filterkuchen, **dadurch gekennzeichnet, dass** er nach einem Verfahren gemäß einem der Ansprüche 1 bis 11 hergestellt wurde, wobei der Gehalt des Filterkuchens an anorganischen Salzen weniger als 25 Gew.-% bezogen auf die Trockenmasse des Filterkuchens beträgt, wobei zur Bestimmung des Salzgehaltes das in der Beschreibung genannte Messverfahren eingesetzt wird.

## Claims

1. Method comprising the steps:
(1) providing a composition (12) containing proteins and amino acids from mucous membranes of slaughtered animals, wherein the mucous membranes of slaughtered animals have been mixed with salts and subjected to enzymatic hydrolysis, inorganic salts and water, wherein the sum of the mass of the proteins and amino acids in the dry mass of this composition is 30 to 70 percent by weight and the mass of the inorganic salts in the dry mass of this composition (12) is at least 7,5 percent by weight, wherein at least a proportion of 50 percent by weight of the inorganic salts consists of the elements sodium and sulphur as well as chloride , wherein the proportion of sodium sulphate in the total amount of inorganic salts is at least 50 percent by weight,
(2) filtering the composition (12) while obtaining a filtrate (14) and a filter cake (15), wherein a filter with a pore size of more than 2 µm is used for filtering the composition (12),
(3) cooling the filtrate (14) to a temperature in the range from -15°C to 15°C while forming a precipitate (19),
(4) separating the resulting precipitate (19) while obtaining the liquid phase (23) as a product, wherein the measuring methods mentioned in the description are used to determine the mass of proteins and amino acids as well as salts.

2. Method according to claim 1, **characterized in that** the composition (12) provided in step (1) contains proteins and amino acids from the intestinal mucosa of slaughtered animals.

3. Method according to one of claims 1 or 2, **characterized in that** the composition (12) provided in step (1) contains proteins and amino acids which are obtained as a waste product during the extraction of heparin from the intestinal mucosa of slaughtered animals.

4. Method according to one of the preceding claims, **characterized in that** a microfiltration system or a chamber filter press (13) is used for filtering the composition (12) in step (2).

5. Method according to one of the preceding claims, **characterized in that** the composition (12) provided in step (1) comprises 25 to 50 % by weight of proteins and amino acids, 30 to 60, preferably 35 to 50, % by weight of water and 7.5 to 30 % by weight of inorganic salts.

6. Method according to one of the preceding claims, **characterized in that** the filtrate (14) obtained in step (2) is cooled to a temperature in the range from - 12.5°C to 12°C, preferably from -10°C to 10°C and particularly preferably from - 5°C to 5°C in step (3) for forming the precipitate (19).

7. Method according to one of the preceding claims, **characterized in that** at least 50 % by weight of the precipitate (19) obtained in step (4) consists of inorganic salts of the elements sodium and sulphur as well as chlorine.

8. Method according to one of the preceding claims, **characterized in that** the separation of the precipitate (19) formed in step (3) is carried out in step (4) by centrifugation.

9. Method according to one of the preceding claims, **characterized in that** the liquid phase (23) obtained in step (4) contains less than 25 % by weight of inorganic salts, particularly preferably less than 20 % by weight of inorganic salts, very particularly preferably less than 17.5 % by weight of inorganic salts relative to the dry mass.

10. Method according to one of the preceding claims, **characterized in that** the filter cake (15) obtained in step (2) contains less than 25 % by weight of inorganic salts, particularly preferably less than 20 % by weight of inorganic salts relative to the dry mass.

11. Method according to one of the preceding claims, **characterized in that** the proportion of inorganic salts in the dry mass of the liquid phase (23) obtained in step (4) and/or in the dry mass of the filter cake (15) obtained in step (2) is reduced by at least 25%, preferably by at least 35% and most preferably by at least 40%, compared to the proportion of inorganic salts in the dry mass of the composition used.

12. Filter cake, **characterized in that** it has been produced by a method according to one of claims 1 to 11, wherein the content of inorganic salts in the filter cake is less than 25% by weight based on the dry mass of the filter cake, wherein the measuring method mentioned in the description is used to determine the salt content.

## Revendications

1. Procédé comprenant les étapes consistant à:
(1) mise à disposition d'une composition (12) contenant des protéines et des acides aminés provenant de muqueuses d'animaux de boucherie, les muqueuses d'animaux de boucherie ayant été mélangées avec des sels et soumises à une hydrolyse enzymatique, des sels inorganiques et de l'eau, la somme de la masse des protéines et des acides aminés dans la masse sèche de cette composition étant de 30 à 70 pour cent en poids et la masse des sels inorganiques dans la masse sèche de cette composition (12) étant d'au moins 7,5 % en poids, au moins une proportion de 50 % en poids des sels inorganiques étant constituée des éléments sodium et soufre et chlorure, la proportion de sulfate de sodium par rapport à la quantité totale des sels inorganiques étant d'au moins 50 % en poids,
(2) filtrer la composition (12) pour obtenir un filtrat (14) et un gâteau de filtration (15), dans lequel on utilise pour filtrer la composition (12) un filtre ayant une taille de pores supérieure à 2 µm,
(3) refroidir le filtrat (14) à une température comprise entre -15°C et 15°C pour former un précipité (19),
(4) séparation du précipité formé (19) avec obtention de la phase liquide (23) en tant que produit, les procédés de mesure mentionnés dans la description étant utilisés pour déterminer la masse de protéines et d'acides aminés ainsi que de sels.

2. Procédé selon la revendication 1, **caractérisé en ce que** la composition (12) fournie à l'étape (1) contient des protéines et des acides aminés provenant de la muqueuse intestinale d'animaux de boucherie.

3. Procédé selon l'une des revendications 1 ou 2, **caractérisé en ce que** la composition (12) préparée à l'étape (1) contient des protéines et des acides aminés qui sont des déchets obtenus lors de l'extraction d'héparine de la muqueuse intestinale d'animaux de boucherie.

4. Procédé selon l'une quelconque des revendications précédentes, **caractérisé en ce que**, pour filtrer la composition (12) à l'étape (2), on utilise une installation de microfiltration ou un filtre-presse à chambres (13).

5. Procédé selon l'une quelconque des revendications précédentes, **caractérisé en ce que** la composition (12) fournie à l'étape (1) comprend de 25 à 50 % en poids de protéines et d'acides aminés, de 30 à 60 %, de préférence de 35 à 50 %, en poids d'eau et de 7,5 à 30 % en poids de sels inorganiques.

6. Procédé selon l'une quelconque des revendications précédentes, **caractérisé en ce que** le filtrat (14) obtenu à l'étape (2) est refroidi à une température comprise entre -12,5°C et 12°C, de préférence entre -10°C et 10°C et plus préférentiellement entre -5°C et 5°C, pour former le précipité (19) à l'étape (3).

7. Procédé selon l'une quelconque des revendications précédentes, **caractérisé en ce qu'**au moins 50 % en poids du précipité (19) obtenu à l'étape (4) sont constitués de sels inorganiques des éléments sodium et soufre et de chlore.

8. Procédé selon l'une quelconque des revendications précédentes, **caractérisé en ce que** la séparation du précipité (19) formé à l'étape (3) est réalisée par centrifugation à l'étape (4).

9. Procédé selon l'une quelconque des revendications précédentes, **caractérisé en ce que** la phase liquide (23) obtenue à l'étape (4) contient moins de 25 % en poids de sels inorganiques, plus préférentiellement moins de 20 % en poids de sels inorganiques, tout particulièrement moins de 17,5 % en poids de sels inorganiques par rapport à la matière sèche.

10. Procédé selon l'une quelconque des revendications précédentes, **caractérisé en ce que** le gâteau de filtration (15) obtenu à l'étape (2) contient moins de 25 % en poids de sels inorganiques, de préférence moins de 20 % en poids de sels inorganiques par rapport à la matière sèche.

11. Procédé selon l'une quelconque des revendications précédentes, **caractérisé en ce que** la proportion de sels inorganiques dans la matière sèche de la phase liquide (23) obtenue à l'étape (4) et/ou dans la matière sèche du gâteau de filtration (15) obtenu à l'étape (2) est réduite d'au moins 25%, de préférence d'au moins 35% et plus préférentiellement d'au moins 40% par rapport à la proportion de sels inorganiques dans la matière sèche de la composition mise en œuvre.

12. Gâteau de filtration, **caractérisé en ce qu'**il a été fabriqué selon un procédé selon l'une des revendications 1 à 11, la teneur du gâteau de filtration en sels inorganiques étant inférieure à 25 % en poids par rapport à la masse sèche du gâteau de filtration, le procédé de mesure mentionné dans la description étant utilisé pour déterminer la teneur en sels.
